(19) Europäisches Patentamt / European Patent Office / Office européen des brevets

(11) **EP 3 563 086 B1**

(12) **FASCICULE DE BREVET EUROPEEN**

(45) Date de publication et mention de la délivrance du brevet:
**23.12.2020 Bulletin 2020/52**

(51) Int Cl.:
***F23D 3/02*** *(2006.01)*    ***A61L 9/03*** *(2006.01)*
***F23D 3/18*** *(2006.01)*

(21) Numéro de dépôt: **17828995.5**

(22) Date de dépôt: **21.12.2017**

(86) Numéro de dépôt international:
**PCT/FR2017/053761**

(87) Numéro de publication internationale:
**WO 2018/122502 (05.07.2018 Gazette 2018/27)**

(54) **BRÛLEUR À COMBUSTION CATALYTIQUE EN MATERIAU POREUX À PERFORMANCES DE FONCTIONNEMENT OPTIMISÉES ET FLACON EQUIPÉ D'UN TEL BRÛLEUR**

AUS EINEM PORÖSEN MATERIAL HERGESTELLTER, KATALYTISCHER VERBRENNER MIT OPTIMIERTER BETRIEBSLEISTUNGEN UND MIT SOLCH EINEM VERBRENNER AUSGESTATTETE FLASCHE

CATALYTIC COMBUSTION BURNER MADE OF POROUS MATERIAL, WITH OPTIMISED OPERATION PERFORMANCE AND BOTTLE EQUIPPED WITH SUCH A BURNER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2016 FR 1663555**

(43) Date de publication de la demande:
**06.11.2019 Bulletin 2019/45**

(73) Titulaire: **Produits Berger**
**27520 Grand Bourgtheroulde (FR)**

(72) Inventeurs:
• **GERARD, Corinne**
  **27400 Incarville (FR)**
• **OZOUF, Laurent**
  **76940 Notre-Dame-de-Bliquetuit (FR)**
• **PAJOT, Laetitia**
  **87920 Condat sur Vienne (FR)**
• **CELLIER, Matthieu**
  **87510 Saint Jouvent (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A1- 1 491 819        FR-A1- 2 779 509**
**FR-A1- 2 905 164        TW-A- 201 215 417**
**US-A1- 2007 190 472**

**Description**

La présente invention concerne de manière générale le domaine de la combustion catalytique, et plus précisément celui des brûleurs à combustion catalytique en matériau poreux. Ces brûleurs sont en particulier utilisés pour la diffusion de parfum et ou de substances actives, pour la destruction de molécules odorantes ou non, et/ou pour l'assainissement de l'air.

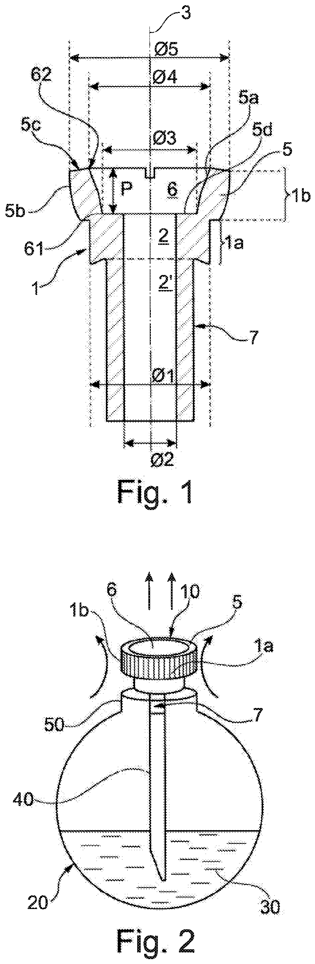

Un tel brûleur a par exemple été décrit dans le brevet FR2610390 au nom de la demanderesse. Il est notamment destiné à recevoir une mèche trempant dans un liquide combustible contenu dans un flacon à combustion catalytique, qui reçoit le brûleur au niveau de son goulot. Un tel brûleur (notamment représenté sur la figure 1 est réalisé en un matériau poreux, qui comprend un embout présentant à sa partie supérieure une cavité débouchant à l'extérieur et à sa partie inférieure, une cavité dans laquelle est engagée l'extrémité de la mèche. L'embout se prolonge à sa partie inférieure par un manchon. En fonctionnement, le liquide combustible amené par la mèche pénètre dans les pores du matériau poreux du brûleur. Une partie de ce liquide traverse la zone centrale du brûleur et y subit une vaporisation.

Par ailleurs, la demanderesse a développé un brûleur céramique à base de SiC, tel que par exemple ceux décrits dans les brevets français FR2095163B1, FR2905164B1 et FR2905165B1 appartenant à la demanderesse, pour équiper les lampes à catalyse. Ce type de brûleur répond à de nombreuses performances en termes de parfumage, consommation, qualité olfactive, émissions... Mais, il présente l'inconvénient de dysfonctionner voire de s'éteindre lorsqu'il est soumis à de forts courants d'air tels que la climatisation. Quelques pays, forts utilisateurs de climatisation, se retrouvent donc confrontés à des problèmes récurrents d'extinction de bruleur lorsqu'ils placent leur lampe à proximité de la climatisation et ne peuvent donc pas utiliser les bruleurs cités précédemment.

Afin de pallier les inconvénients précités, la demanderesse a mis au point un brûleur à combustion catalytique en matériau poreux selon l'objet de la revendication 1.

Le catalyseur utilisé dans le cadre de la présente invention peut être par exemple un catalyseur à base d'un métal appartenant aux groupes 9 ou 10 du Tableau de Classification périodique des éléments (selon la terminologie recommandée par l'UICPA).

L'insert peut être réalisé dans le même matériau poreux que le brûleur. Il peut aussi être réalisé en un autre matériau poreux.

Avec une telle composition, le matériau poreux du bruleur selon l'invention présente une porosité comprise entre 57% et 63%, et de préférence de l'ordre de 59%, et des interconnexions entre 8 et 11 $\mu$m.

Par interconnexions, on entend, au sens de la présente invention le diamètre médian des interconnexions poreuses.

De manière avantageuse, une partie au moins de la face intérieure de la paroi périphérique latérale peut également être dopée par ledit catalyseur. Cela permet d'améliorer encore les performances en fonctionnement en présence d'un climatiseur ou d'une ventilation, comme le montrent les exemples ci-après.

De manière avantageuse, la profondeur P de la deuxième cavité peut être comprise entre 2 et 8 mm, de préférence entre 6 et 7,5 mm et mieux de l'ordre de 7 mm.

De manière avantageuse, l'extrémité inférieure de la face intérieure peut se terminer par un lamage de diamètre $\phi_3$, communiquant avec la première cavité.

Par lamage, on entend, au sens de la présente invention, un usinage cylindrique réalisé autour d'un perçage communiquant avec la première cavité de la partie inférieure de l'embout.

Grâce à la présence du lamage ainsi qu'à la nature et à la porosité du matériau poreux constitutif du brûleur, les performances de fonctionnement en présence d'une climatisation sont encore améliorées.

De manière avantageuse, le diamètre extérieur $\phi_1$ de la partie inférieure de l'embout peut être compris entre 14 et 17 mm, de préférence entre 15 et 16 mm, et mieux de l'ordre de 15,6 mm.

De manière avantageuse, le matériau poreux est obtenu à partir d'une composition comprenant en pourcentage du poids total de ladite composition, comprenant en pourcentage du poids total de ladite composition, entre 0,5 et 2% dudit composé conducteur thermique, entre 40 et 70% dudit composé réfractaire, entre 5 et 25 % dudit liant, et entre 8 et 35 % d'au moins un agent porogène.

La composition du matériau poreux selon l'invention comprend notamment un composé conducteur thermique et un composé réfractaire.

A titre de composé conducteur thermique utilisable dans le matériau poreux du bruleur selon l'invention, on pourra avantageusement utiliser du carbure de silicium, de préférence présent à raison de 1% en poids par rapport au poids total de ladite composition. Le pourcentage de composé conducteur thermique (entre 0 et 5%, et de préférence de l'ordre de 1%) est optimisé de manière à obtenir des caractéristiques de fonctionnement idéales.

A titre de composé réfractaire, on utilisera de préférence un composé réfractaire présentant une conductivité thermique inférieure à la conductivité thermique dudit conducteur thermique. De manière avantageuse, ledit composé réfractaire peut être choisi dans le groupe constitué de l'alumine, la silice, la mullite, la zircone, la cordiérite, et leurs mélanges. Le matériau réfractaire est de préférence la mullite. La mullite peut par exemple être remplacée par ou mélangée à l'alumine, la silice, la zircone, la cordiérite, ou un de leurs mélanges. La silice est de préférence introduite dans la composition de l'invention en mélange avec un autre composé réfractaire. Le composé réfractaire, en plus de son bon comportement mécanique à haute température, joue un rôle principal d'isolant au sein du matériau poreux obtenu à partir de la composition selon l'invention.

Outre le composé conducteur thermique et composé réfractaire, la composition du matériau poreux selon l'invention comprend un liant et un agent porogène.

Par liant, on entend au sens de la présente un composé minéral permettant un frittage à une température inférieure ou égale à 1100°C.

Le liant est de manière avantageuse un verre présentant un pourcentage en silice variable, plus particulièrement un verre ordinaire à base d'oxydes, par exemple incluant environ 70% de silice et environ 30% d'oxydes de calcium et de sodium, ou un verre spécial incluant des oxydes d'éléments divers tels que bore ou phosphate, par exemple un verre borosilicaté.

Le liant améliore l'aptitude à la mise en forme, apporte la cohésion mécanique en cru d'une pièce et permet d'obtenir un matériau poreux et résistant mécaniquement. Le liant est choisi en particulier parmi les composés de frittage à basse température, c'est à dire les composés permettant le frittage de la composition dans laquelle ils interviennent à cette température, inférieure ou égale à 1100°C. Par exemple, lors de la montée en température lors du frittage de la composition de l'invention, le verre se ramollit et mouille les particules de composé conducteur thermique. Une phase vitreuse est ainsi obtenue. Lors, ensuite, de la descente en température, les particules sont collées entre elles lors de la solidification de la phase vitreuse.

A titre d'agent porogène utilisable dans le matériau poreux du bruleur selon l'invention, on pourra avantageusement choisir tout composé porogène (naturel ou de synthèse), dont la granulométrie est maîtrisée et contrôlée, notamment au cours de sa fabrication et de son stockage. Par exemple, on pourra avantageusement utiliser à titre d'agent porogène le polyméthacrylate de méthyle (PMMA). Celui si pourra avantageusement être présent à raison de 18 à 30% en poids par rapport au poids total de ladite composition. L'agent porogène permet la formation de pores au cours du frittage de la composition selon l'invention.

Ainsi, de manière préférentielle, le matériau poreux pourra être réalisé à partir d'une composition de l'ordre de 1% de carbure de silicium, entre 60 et 70 % de mullite, entre 5 et 15 % de verre, et de l'ordre de 18 à 30 % de PMMA.

Avec une telle composition, le matériau poreux du brûleur selon l'invention présente une porosité de l'ordre de 60%, et des interconnexions de l'ordre de 9,5 $\mu$m.

De manière avantageuse, ledit manchon du bruleur peut présenter une longueur comprise entre 10 et 20 mm, et de préférence de l'ordre de 14 mm. Comme illustré dans les exemples, il est possible d'atteindre de bonnes performances en termes de résistance à la climatisation, avec un brûleur selon l'invention présentant une longueur de manchon pouvant être aussi faible que 12 mm.

La présente invention a encore pour objet un flacon à combustion catalytique, adapté à contenir un liquide combustible et à recevoir au niveau de son goulot un brûleur à combustion catalytique recevant une mèche trempant dans ledit liquide, caractérisé en ce que ledit flacon est équipé d'un brûleur selon l'invention tel que défini précédemment.

D'autres caractéristiques et avantages de l'invention ressortiront clairement de la description détaillée qui en est faite ci-après, à titre indicatif et nullement limitatif, en référence aux dessins annexés dans lesquels :

- la figure 1 représente schématiquement une coupe transversale d'un premier exemple de brûleur utilisable dans le cadre de la présente invention ;
- la figure 2 est une vue schématique en élévation d'un flacon équipé de l'un des brûleurs à combustion représentés sur la figure 1 d'une part et sur les figures 5 et 6 d'autre part ;
- les figures 3 et 4 représentent respectivement une vue en perspective latérale et une vue de dessus d'un insert utilisable dans le brûleur selon l'invention.
- la figure 5 représente schématiquement une coupe transversale d'un deuxième exemple de brûleur utilisable dans le cadre de la présente invention ;
- la figure 6 représente une vue en perspective du brûleur illustré sur la figure 5 ;
- les figures 7 à 18 sont des thermographies IR réalisées pour montrer l'impact d'une climatisation sur un brûleur selon l'invention d'une part et sur un brûleur témoin ne présentant pas de catalyseur sur la zone annulaire 5c ;
- La figure 19 illustre le protocole de mesure par caméra infrarouge de l'impact d'une climatisation sur la température du brûleur en fonctionnement.

Les caractéristiques techniques communes à ces deux figures sont désignées chacune par la même référence numérique dans les figures concernées.

Sur les figures 1, 5 et 6, sont représentés deux exemples de brûleurs 10 utilisables dans le cadre de la présente invention. Ces deux exemples de brûleur 10 comprennent chacun d'une part un embout 1 avec une partie inférieure 1a et une partie supérieure 1b, et d'autre part un manchon 7 disposé dans le prolongement de la de partie inférieure 1a de

l'embout 1.

En ce qui concerne plus particulièrement l'embout 1, celui-ci comporte :

- la partie inférieure la de diamètre extérieur $\phi_1$ et délimitant une première cavité 2 de diamètre $\phi_2$, cette première cavité 2, qui s'étend le long d'un axe principal 3, étant adaptée à recevoir une mèche propre à imbiber l'embout 1 d'une composition combustible, et
- une partie supérieure 1b présentant une paroi périphérique latérale 5 comportant une face intérieure 5a de forme essentiellement tronconique et délimitant une deuxième cavité 6 de profondeur P, une face extérieure 5b de forme essentiellement cylindrique (mais tronconique au niveau de sa base) et de diamètre $\phi_5$, une face supérieure 5c et un fond 5d communiquant avec la première cavité.

La face intérieure 5a présente une extrémité inférieure 61 de diamètre $\phi_3$ supérieur à $\phi_2$ et une extrémité supérieure 62 de diamètre $\phi_4$ supérieur à $\phi_3$, l'extrémité supérieure 62 de la paroi latérale 5 communiquant avec l'atmosphère et l'extrémité inférieure 61 étant reliée au fond 5d. Les première 2 et deuxième 6 cavités communiquent ensemble.

En ce qui concerne plus particulièrement le manchon (également appelé fût) 7, celui-ci est disposé dans le prolongement de la partie inférieure la de l'embout 1. Il délimite une troisième cavité 2' prolongeant la première cavité 2 de la partie inférieure la. Ce manchon est constitué du même matériau poreux que l'embout.

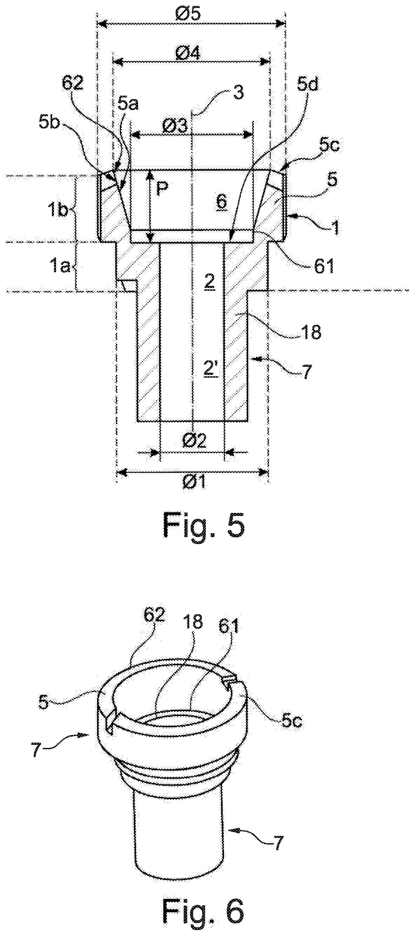

Les figures 5 et 6 montrent respectivement schématiquement une coupe transversale schématique et une vue en perspective d'un deuxième exemple de brûleur, qui se différencie du premier exemple de brûleur en ce que, dans le deuxième exemple de brûleur, l'extrémité inférieure 61 de la face intérieure 5a se termine par un lamage 18 de diamètre $\phi_3$, communiquant avec la cavité 2. Dans le premier exemple de brûleur représenté sur la figure 1 (sans lamage), la face intérieure 5a est entièrement tronconique entre ses deux extrémités 61 et 62.

Les deux exemples de brûleurs (représentés sur les figures 1, 5 et 6) comportent chacun un insert 8 disposé dans la deuxième cavité 6 de l'embout 1 et présentant une surface 8a en contact avec ledit fond 5d de l'embout.

En outre, une partie au moins de leur face extérieure 5b est dopée par un catalyseur, par exemple à base d'un métal appartenant aux groupes 9 ou 10 du Tableau de Classification périodique des éléments (selon la terminologie recommandée par l'UICPA).

Lorsque le premier exemple de brûleur ne comporte pas de catalyseur sur sa face 5c, il est utilisé à titre de témoin dans les essais de comportement à la climatisation (désigné ci-après par la référence 1C).

Lorsque le premier exemple de brûleur ne comporte pas catalyseur dopant sa face 5c, il est utilisé à titre de premier exemple de brûleur selon l'invention dans les essais de comportement à la climatisation (désigné ci-après par la référence 1).

Le deuxième exemple de brûleur comporte un catalyseur dopant sa face 5c, et est utilisé à titre de deuxième exemple de brûleur selon l'invention dans les essais de comportement à la climatisation (désigné ci-après par la référence 2).

Afin de tester le fonctionnement catalytique en présence d'une climatisation des bruleurs représentés sur les figures 1, 5 et 6, ceux-ci ont été disposés dans un flacon 20 à combustion catalytique, représenté sur la figure 2.

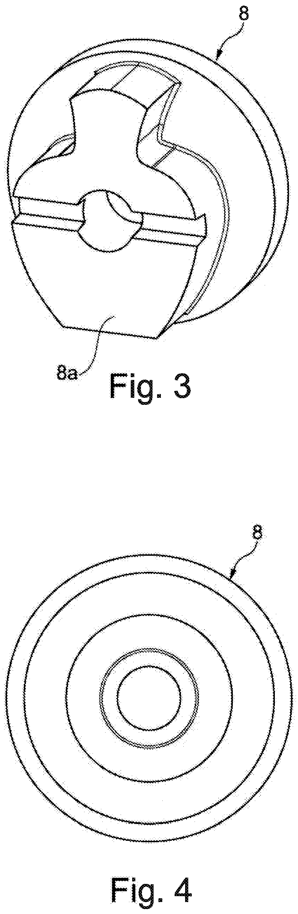

Un tel flacon 20 contient en fonctionnement un liquide combustible 30. Le brûleur 10 (soit celui selon l'invention tel que représenté sur la figure 5, soit celui de l'art antérieur représenté sur la figure 1, munis chacun d'un insert tel que représenté sur la figure 3) est installé dans le goulot 50 du flacon (par exemple à l'aide d'une embase métallique placée dans le goulot 50). Une mèche 40 est reçue à l'intérieur des cavités 2 et 2' cavités du brûleur 10, cette mèche 40 à combustion catalytique recevant une mèche (40) trempant dans le liquide 30. Le flacon 20 peut être un flacon de forme quelconque présentant un goulot 50 dans lequel est adapté le brûleur 10. Le liquide combustible 30 est habituellement un alcool, par exemple de l'alcool isopropylique, ou tout autre liquide combustible approprié compatible avec la législation en vigueur dans ce domaine. En particulier, ce combustible doit être tel que sa vaporisation et sa combustion catalytique ne dégagent pas d'odeur désagréable. Le liquide combustible 30 peut en outre comprendre une matière parfumée et/ou une matière active. La mèche 40 est une mèche connue quelconque, par exemple une mèche en coton, ou une mèche en matière minérale, par exemple en fibres minérales.

En fonctionnement, le liquide combustible 30 du flacon 20 monte dans la mèche 40 par capillarité et pénètre dans les pores de la matière poreuse du brûleur, qui lorsqu'il a été préchauffé, assure sa combustion catalytique.

Les exemples suivants illustrent l'invention, en liaison avec les figures, sans toutefois en limiter la portée.

Dans ces exemples, sauf indication contraire, tous les pourcentages et parties sont exprimés en pourcentages massiques.

EXEMPLES

Composés entrant dans la composition des matériaux poreux utilisés :

- composé conducteur thermique : carbure de silicium,

- composé réfractaire : mullite,

- liant : verre,

- agent porogène : polyméthacrylate de méthyle (PMMA).

Compositions du matériau poreux :

Les brûleurs utilisés dans les exemples sont réalisés par pressage à sec à partir des compositions suivantes C1 et C2 indiquées dans le tableau 1. Pour chacune de ces compositions, nous avons indiqué dans le tableau 1 la porosité de la structure céramique et le diamètre médian des interconnexions.

Tableau 1

| Composition | Mullite (%) | SiC (%) | Verre (%) | PMMA (%) | Porosité (%) | Diamètre d'interconnexions ($\mu$m) |
|---|---|---|---|---|---|---|
| C1 | 64 | 5 | 10 | 21 | 58 à 60% à 975°C | 9 |
| C2 | 66,5 | 1 | 11,5 | 21 | 60,2 à 1050°C | 9,5 |
| C3 | 67 | 0,5 | 11,5 | 21 | 58,9 | - |

Catalyseurs

Le catalyseur utilisé (que ce soit sur les parties 5a, 5b et 5C du brûleur) est un métal appartenant aux groupes 9 ou 10 du tableau de classification périodique des éléments.

**Brûleurs utilisés** :

A titre d'exemple comparatif :

Brûleur 1C (représenté sur la figure 1), dont la face extérieure 5b est dopée par un catalyseur l'imprégnant, le brûleur 1C étant constitué d'un matériau poreux obtenu à partir de la composition C1.

A titre d'exemples selon l'invention :

- brûleur 1 (également représenté sur la figure 1 et comprenant l'insert représenté sur les figures 3 et 4) identique au brûleur 1C, hormis sa face 5c qui est dopée par le même catalyseur que celui de la face extérieure 5b, le brûleur 1 étant également constitué d'un matériau poreux obtenu à partir de la composition C1.
- brûleur 2 (représenté sur les figures 5 et 6 et comprenant l'insert représenté sur les figures 3 et 4), dont la face extérieure 5b et la face supérieure 5c sont dopées par le même catalyseur qui les imprègnent, le brûleur 2 étant constitué d'un matériau poreux obtenu à partir de la composition C2.
- brûleur 3 (représenté sur les figures 5 et 6 et comprenant l'insert représenté sur les figures 3 et 4), dont la face extérieure 5b et la face supérieure 5c sont dopées par le même catalyseur qui les imprègnent, le brûleur 3 étant constitué d'un matériau poreux obtenu à partir de la composition C3.

En fonctionnement, lorsque le brûleur est muni d'un catalyseur dans sa partie périphérique annulaire 5c, la partie du liquide combustible qui parvient dans cette partie 5c y subit une combustion catalytique qui maintient cette partie à une température élevée.

**Flacon utilisé** : celui représenté sur la figure 2 pour les brûleurs 1C, 1 et 2.

**Mèche utilisée** : mèches en coton (pour le brûleur 3, deux mèches différentes ont été testées).

**Liquide combustible utilisé** : alcool isopropylique.

**Tests et mesures**

1) Porosité (%) et Diamètre D des interconnexions

On mesure la porosité ouverte du matériau poreux constitutif du brûleur par intrusion de mercure dans le matériau d'un porosimètre de marque MICROMERITICS AUTOPORE IV 9510. Cette mesure est réalisée à une pression maximale de 414 MPa environ, ce qui correspond à une taille minimale des pores détectables de l'ordre de 0,0035pm.

Méthode et protocole opératoire :

La mesure de la porosité par intrusion de mercure est basée sur le principe de pénétration d'un liquide non réactif dans un matériau poreux, en immergeant celui-ci dans le liquide et en augmentant la pression de manière isostatique. Le mercure, n'ayant pas de réaction avec la plupart des matériaux, est de plus un liquide idéal du fait de la valeur élevée de son angle de contact, il ne mouille pas la plupart des matériaux.

A partir de cette mesure, on détermine la taille des pores en terme de diamètre D en $\mu$m (diamètre des interconnexions), alors pénétré, est inversement proportionnelle à la pression appliquée, P, d'après l'équation de WASBURN :

$$D = \frac{-4\gamma \cos \Theta}{P}$$

Avec :

$\gamma$ : tension superficielle du mercure,
$\gamma$ = 0,00485 N/cm (485 dynes/cm).
$\theta$ : angle de contact du mercure, $\theta$ = 140°

2) Caractéristiques de fonctionnement des brûleurs 10 installés sur le flacon 20 en présence d'un climatiseur à 18°C avec ventilation.

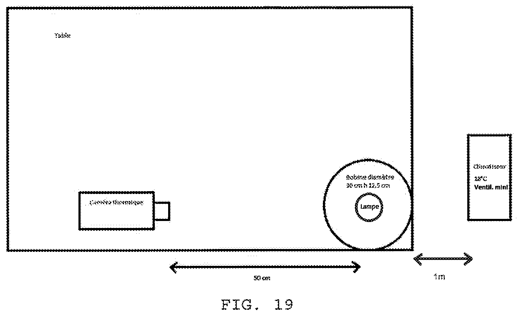

Le protocole d'essais est représenté sur la figure 19. Il consiste globalement à mesurer par thermographie infrarouge (IR) à l'aide d'une caméra thermique IR la température sur chacun des brûleurs testés, placé à distance raisonnable d'un climatiseur (dont la puissance est de 800 W dans le cadre des essais réalisés). Ces mesures sont comparées, pour chaque brûleur testé (témoin 1C et selon l'invention 1 et 2), à des mesures réalisées sans ventilation.
Les thermographies réalisées sont détaillées ci-après :

- Brûleur 1C :

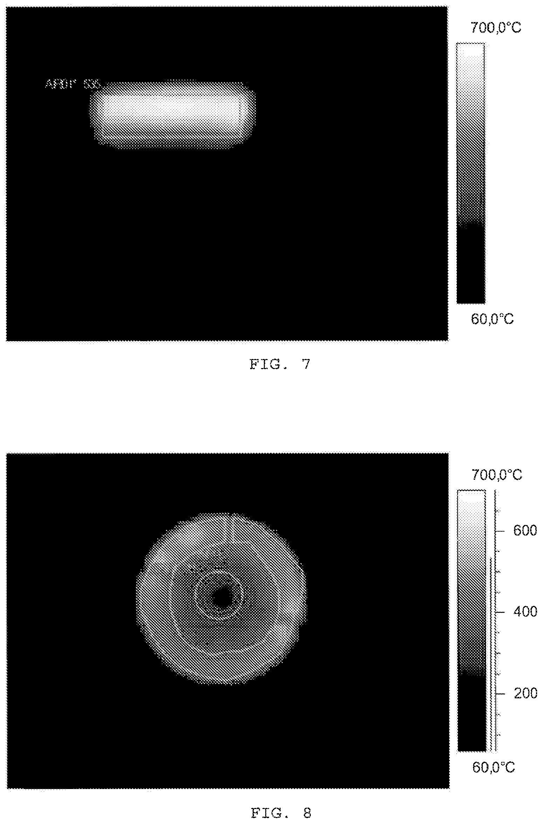

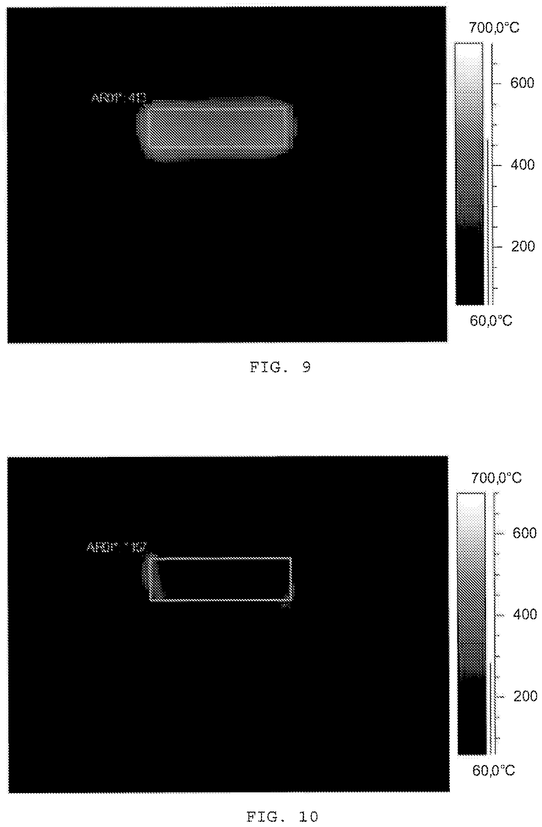

   • Sans climatisation : figures 7 et 8 ;
   • Avec climatisation : figure 9 (mesure réalisée du côté opposé à la climatisation) et figure 10 (mesure réalisée du côté face à la climatisation) ;

- Brûleur 1 :

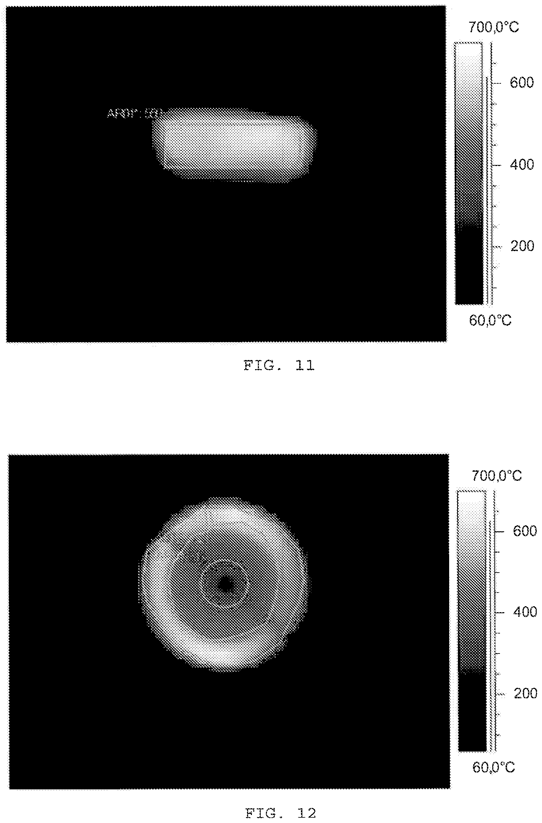

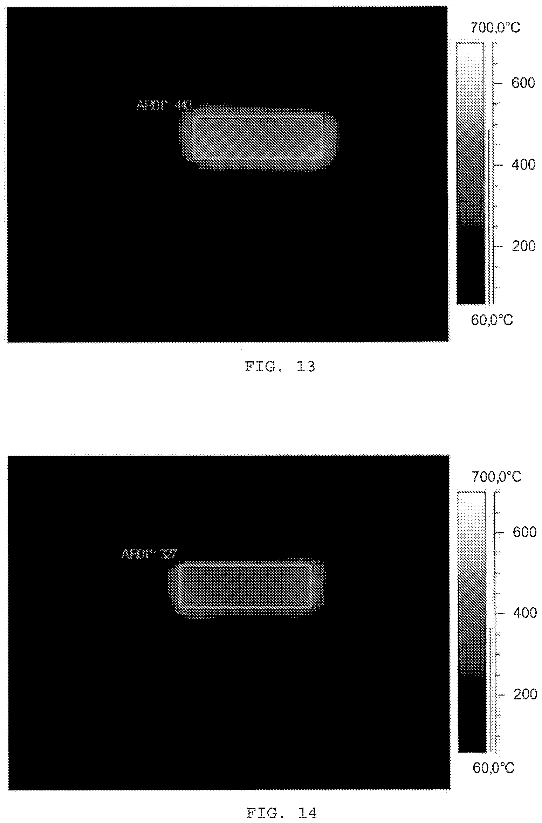

   • Sans climatisation : figures 11 et 12 ;
   • Avec climatisation : figure 13 (mesure réalisée du côté opposé à la climatisation) et figure 14 (mesure réalisée du côté face à la climatisation) ;

- Brûleur 2 :

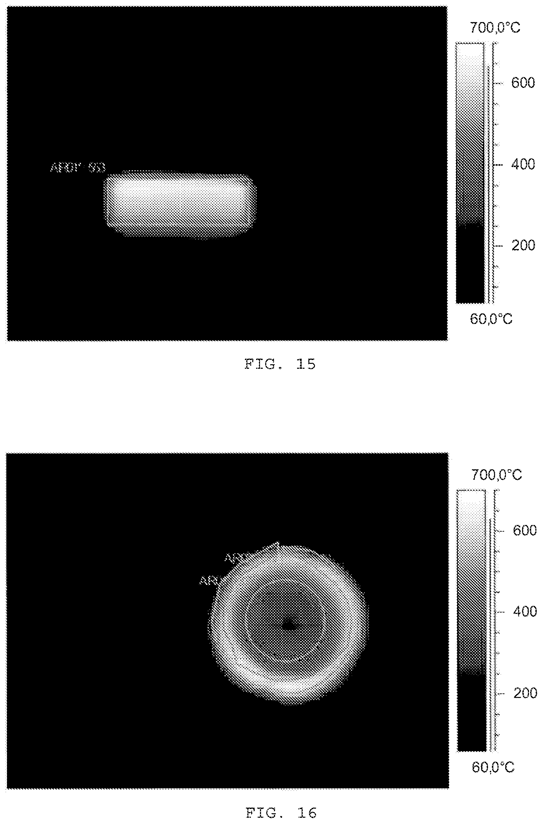

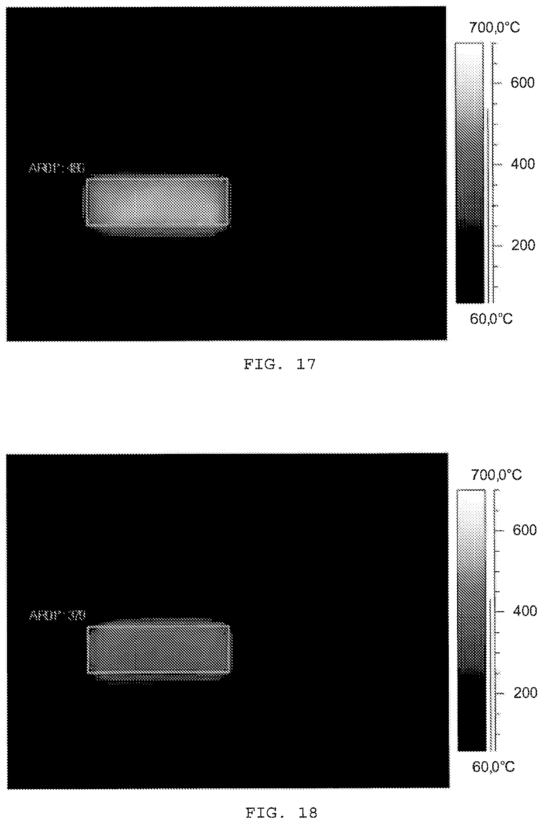

   • Sans climatisation : figures 15 et 16 ;
   • Avec climatisation : figure 17 (mesure réalisée du côté opposé à la climatisation) et figure 18 (mesure réalisée du côté face à la climatisation) ;

Lorsque la mesure est réalisée du côté du brûleur qui est opposé au flux d'air provenant du climatiseur, ce dernier a un peu d'impact sur la température mesurée sur ce côté du brûleur, comme le montrent la comparaison des figure 7 et 9 (brûleur 1C), les figures 11 et 13 (brûleur 1 selon l'invention), et les figures 15 et 17 (brûleur 1 selon l'invention). Cette température diminue très peu pour les brûleurs selon l'invention 1 et 2, et un peu plus pour le brûleur témoin 1.

Il n'en est pas de même lorsque la mesure est réalisée du côté du brûleur faisant face au climatiseur : les brûleurs selon l'invention 1 et 2 montrent une meilleure résistance que le brûleur témoin 1C, comme le montrent la comparaison des figures 7 et 10 (chute drastique pour le brûleur témoin), celle des figures 11 et 14, et enfin celle des figures 15 et 18. On considère que lorsque la température mesurée à un endroit donné du catalyseur est inférieure à 300°C, celui-ci a de grandes chances de s'éteindre rapidement.

Les températures mesurées sont rassemblées dans le tableau 2 ci-après :

Tableau 2

| | Mesure de la température sans climatisation | | | Mesure de la température avec climatiseur | |
|---|---|---|---|---|---|
| | Côté | Dessus | | Côté opposé climatiseur | Côté face climatiseur |
| | Face 5b | Face 5c | Centre | | |
| Bruleur 1C | 535°C | 460°C | 283°C | 413°C | 157°C |
| Bruleur 1 | 559°C | 521°C | 313°C | 443°C | 327°C |
| Bruleur 2 | 553°C | 537°C | 339°C | 486°C | 379°C |
| Bruleur 3 (essai mèche 1) | 546°C | 559°C | 347°C | 483°C | 404°C |
| Bruleur 3 (essai mèche 2) | 556°C | 532°C | 336°C | 523°C | 411°C |

Ces essais montrent que la présence d'un catalyseur sur la zone annulaire 5c du brûleur permet de maintenir la température du brûleur quand il est soumis à un fort courant d'air, tel que par exemple celui émis par un climatiseur portatif, et ce tant pour une teneur en SiC de 1% que pour une teneur en SiC de 0,5%. Ce catalyseur permet une conduction thermique de la chaleur vers la zone du brûleur, qui lorsqu'elle est soumise à un courant d'air, ne se désamorce finalement pas.

## Revendications

1. Brûleur (10) à combustion catalytique en matériau poreux comprenant :

   - un embout (1) comportant :

     • une partie inférieure (1a) de diamètre extérieur $\phi_1$ et délimitant une première cavité (2) de diamètre $\phi_2$, ladite première cavité (2) s'étendant le long d'un axe principal (3) et étant adaptée à recevoir une mèche propre à imbiber l'embout (1) d'une composition combustible, et
     • une partie supérieure (1b) présentant une paroi périphérique latérale (5) comportant une face intérieure (5a) de forme essentiellement tronconique et délimitant une deuxième cavité (6) de profondeur P, une face extérieure (5b), une face supérieure (5c) de forme annulaire et un fond (5d), ladite face intérieure (5a) présentant une extrémité inférieure (61) de diamètre $\phi_3$ supérieur à $\phi_2$ et une extrémité supérieure (62) de diamètre $\phi_4$ supérieur à $\phi_3$, l'extrémité supérieure (62) de ladite paroi latérale (5) communiquant avec l'atmosphère et la partie inférieure (61) dudit fond (d) communiquant avec ladite première cavité (2),
     • une partie au moins de ladite face extérieure (5b) de ladite paroi périphérique latérale (5) est dopée par un catalyseur,

   - un manchon (7) disposé dans le prolongement de ladite partie inférieure (1a) de l'embout (1) et délimitant une troisième cavité (2') prolongeant ladite première cavité (2) de ladite partie inférieure (Ia), et
   - un insert (8) disposé dans ladite deuxième cavité (2) de l'embout (1) et présentant une surface (8a) en contact avec ledit fond (5d) de l'embout,

   ledit brûleur étant **caractérisé en ce que** ledit matériau poreux est obtenu à partir d'une composition comprenant en pourcentage du poids total de ladite composition, entre 0 et 5% d'au moins un composé conducteur thermique, de conductivité thermique supérieure à 60 watts par mètre-kelvin, entre 30 et 70% d'au moins un composé réfractaire, entre 2 et 30 % d'au moins un liant, et entre 5 et 40 % d'au moins un agent porogène, et **en ce que** la totalité de ladite face supérieure (5c) de forme annulaire est dopée par ledit catalyseur.

**2.** Brûleur (10) selon la revendication 1, dans lequel une partie au moins de ladite face intérieure (5a) de ladite paroi périphérique latérale (5) est dopée par ledit catalyseur.

**3.** Brûleur (10) selon les revendications 1 ou 2, dans lequel l'extrémité inférieure (61) de ladite face intérieure (5a) se termine par un lamage (18) de diamètre de diamètre $\phi_3$, communiquant avec ladite cavité (2).

**4.** Brûleur (10) selon l'une quelconque des revendications précédentes, dans lequel la profondeur P de ladite cavité (6) est comprise entre 2 et 8 mm, de préférence entre 6 et 7,5 mm et mieux de l'ordre de 7 mm.

**5.** Brûleur (10) selon l'une quelconque des revendications 1 à 4, selon lequel ledit matériau poreux est obtenu à partir d'une composition comprenant en pourcentage du poids total de ladite composition, entre 0,5 et 2% dudit composé conducteur thermique, entre 40 et 70 % dudit composé réfractaire, entre 5 et 25 % dudit liant, et entre 8 et 35 % d'au moins un agent porogène.

**6.** Brûleur (10) selon l'une quelconque des revendications 1 à 5, dans lequel le composé conducteur thermique est du carbure de silicium, de préférence présent à raison de 1% en poids par rapport au poids total de ladite composition.

**7.** Brûleur (10) selon l'une quelconque des revendications 1 à 6, dans lequel chaque ledit composé réfractaire est choisi dans le groupe constitué de l'alumine, la silice, la mullite, la zircone, la cordiérite, et leurs mélanges, et de préférence la mullite

**8.** Brûleur (10) selon l'une quelconque des revendications 1 à 7, dans lequel ledit liant est un composé minéral permettant un frittage à une température inférieure ou égale à 1100 °C, de préférence un verre, et mieux un verre borosilicaté.

**9.** Brûleur (10) selon l'une quelconque des revendications 1 à 8, dans lequel l'agent porogène est le polyméthacrylate de méthyle (PMMA), qui est de préférence présent à raison de 18% à 30% en poids par rapport au poids total de ladite composition.

**10.** Brûleur (10) selon l'une quelconque des revendications 6 à 9, selon lequel ledit matériau poreux est obtenu à partir d'une composition comprenant en pourcentage du poids total de ladite composition, de l'ordre de 1% de carbure de silicium, entre 60 et 70 % de mullite, entre 5 et 15 % de verre, et entre 18 et 30 % de PMMA.

**11.** Brûleur (10) selon la revendication 10, dans lequel ledit manchon (7) présente une longueur comprise entre 10 et 20 mm, et de préférence de l'ordre de 14 mm.

**12.** Flacon (20) à combustion catalytique, adapté à contenir un liquide combustible (30) et à recevoir au niveau de son goulot (50) un brûleur (10) à combustion catalytique recevant une mèche (40) trempant dans ledit liquide (30), **caractérisé en ce que** ledit flacon (20) est équipé d'un brûleur (10) tel que défini selon l'une quelconque des revendications 1 à 11.


**Patentansprüche**

**1.** Brenner (10) mit katalytischer Verbrennung aus einem porösen Material, umfassend:

- ein Endstück (1), Folgendes beinhaltend:

• ein Unterteil (1a) im Außendurchmesser $\phi_1$ und einen ersten Hohlraum (2) im Durchmesser $\phi_2$ begrenzend, wobei sich der erste Hohlraum (2) entlang einer Hauptachse (3) erstreckt und ausgeführt ist, um einen Docht zu empfangen, der geeignet ist, das Endstück (1) mit einer brennbaren Zusammensetzung zu durchtränken, und
• ein Oberteil (1b), das eine seitliche Umfangswand (5) aufweist, die eine Innenseite (5a) in einer im Wesentlichen kegelstumpfförmigen Form beinhaltet, und einen zweiten Hohlraum (6) mit der Tiefe P begrenzt, eine Außenseite (5b), eine Oberseite (5c) in Ringform und einen Boden (5d), wobei die Innenseite (5a) ein unteres Ende (61) im Durchmesser $\phi_3$, größer als $\phi_2$, und ein oberes Ende (62) im Durchmesser $\phi_4$, größer als $\phi_3$, aufweist, wobei das obere Ende (62) der Seitenwand (5) mit der Atmosphäre kommuniziert, und der untere Teil (61) des Bodens (d) mit dem ersten Hohlraum (2) kommuniziert,
• mindestens ein Teil der Außenseite (5b) der seitlichen Umfangswand (5) mit einem Katalysator dotiert ist,

- eine Muffe (7), die in der Verlängerung des Unterteils (1a) des Endstücks (1) angeordnet ist, und einen dritten Hohlraum (2') begrenzt, der den ersten Hohlraum (2) des Unterteils (1a) verlängert, und
- einen Einsatz (8), der in dem zweiten Hohlraum (2) des Endstücks (1) angeordnet ist, und eine Oberfläche (8a) in Kontakt mit dem Boden (5d) des Endstücks aufweist,

wobei der Brenner **dadurch gekennzeichnet ist, dass** das poröse Material aus einer Zusammensetzung erhalten wird, die in Gesamtgewichtsprozenten der Zusammensetzung zwischen 0 und 5 % mindestens einer Wärmeleiterverbindung, mit einer Wärmeleitfähigkeit von mehr als 60 Watt je Meter-Kelvin, zwischen 30 und 70 % mindestens einer feuerfesten Verbindung, zwischen 2 und 30% mindestens eines Bindemittels und zwischen 5 und 40 % mindestens einen Schäumungsmittels umfasst, und
dadurch, dass die Gesamtheit der Oberseite (5c) in Ringform durch den Katalysator dotiert ist.

2. Brenner (10) nach Anspruch 1, wobei mindestens ein Teil der Innenseite (5a) der seitlichen Umfangswand (5) durch den Katalysator dotiert ist.

3. Brenner (10) nach den Ansprüchen 1 oder 2, wobei das untere Ende (61) der Innenseite (5a) mit einer Durchmessersenkung (18) im Durchmesser $\phi_3$ abschließt, die mit dem Hohlraum (2) kommuniziert.

4. Brenner (10) nach einem der vorstehenden Ansprüche, wobei die Tiefe P des Hohlraums (6) zwischen 2 und 8 mm, vorzugsweise zwischen 6 und 7,5 mm enthalten ist, und besser in der Größenordnung von 7 mm liegt.

5. Brenner (10) nach einem der Ansprüche 1 bis 4, wobei das poröse Material aus einer Zusammensetzung erhalten wird, die in Gesamtgewichtsprozenten der Zusammensetzung zwischen 0,5 und 2 % der Wärmeleiterverbindung, zwischen 40 und 70 % der feuerfesten Verbindung, zwischen 5 und 25 % des Bindemittels und zwischen 8 und 35 % des mindestens einen Schäumungsmittels umfasst.

6. Brenner (10) nach einem der Ansprüche 1 bis 5, wobei die Wärmeleiterverbindung Siliziumkarbid ist, das vorzugsweise mit 1 Gew.-% in Bezug auf das Gesamtgewicht der Zusammensetzung vorhanden ist.

7. Brenner (10) nach einem der Ansprüche 1 bis 6, wobei jede feuerfeste Verbindung aus der Gruppe ausgewählt ist, die aus Aluminiumoxid, Silizium, Mullit, Zirkon, Cordierit und deren Gemischen besteht, und vorzugsweise Mullit.

8. Brenner (10) nach einem der Ansprüche 1 bis 7, wobei das Bindemittel eine Mineralverbindung ist, die ein Sintern bei einer Temperatur kleiner oder gleich 1100 °C erlaubt, vorzugsweise ein Glas, und besser ein feuerfestes Glas.

9. Brenner (10) nach einem der Ansprüche 1 bis 8, wobei das Schäumungsmittel Methylpolymethacrylat (PMMA) ist, das vorzugsweise mit 18 Gew.-% bis 30 Gew.-% in Bezug auf das Gesamtgewicht der Zusammensetzung vorhanden ist.

10. Brenner (10) nach einem der Ansprüche 6 bis 9, wobei das poröse Material aus einer Zusammensetzung erhalten wird, die in Gesamtgewichtsprozenten der Zusammensetzung in der Größenordnung von 1 % Siliziumkarbid, zwischen 60 und 70 % Mullit, zwischen 5 und 15 % Glas und zwischen 18 und 30 % PMMA umfasst.

11. Brenner (10) nach Anspruch 10, wobei die Muffe (7) eine Länge aufweist, die zwischen 10 und 20 mm enthalten ist, und vorzugsweise in der Größenordnung von 14 mm.

12. Flasche (20) mit katalytischer Verbrennung, die ausgeführt ist, um eine brennbare Flüssigkeit (30) zu enthalten und im Bereich ihres Halses (50) einen Brenner (10) mit katalytischer Verbrennung zu empfangen, der einen Docht (40) empfängt, der in die Flüssigkeit (30) eingetaucht ist, **dadurch gekennzeichnet, dass** die Flasche (20) mit einem Brenner (10) nach einem der Ansprüche 1 bis 11 ausgerüstet ist.

**Claims**

1.  Catalytic combustion burner (10) made of porous material, comprising:

    - an end piece (1) comprising:

       • a lower part (1a) of outside diameter $\phi_1$ and delimiting a first cavity (2) of diameter $\phi_2$, said first cavity (2) extending along a main axis (3) and being adapted to receive a wick that is capable of soaking the end piece (1) with a combustible composition, and
       • an upper part (1b) having a peripheral side wall (5) comprising an inner face (5a) of essentially frustoconical shape and delimiting a second cavity (6) of depth P, an outer face (5b), an upper face (5c) of circular shape and a base (5d), said inner face (5a) having a lower end (61) of diameter $\phi_3$ greater than $\phi_2$ and an upper end (62) of diameter $\phi_4$ greater than $\phi_3$, the upper end (62) of said side wall (5) communicating with the atmosphere and said lower end (61) of said base (5d) communicating with said first cavity (2),
       • at least a part of said outer face (5b) of said peripheral side wall (5) is doped with a catalyst,

       - a sleeve (7) arranged in the extension of said lower part (1a) of the end piece (1) and delimiting a third cavity (2') extending said first cavity (2) of said lower part (1a), and
       - an insert (8) arranged in said second cavity (2) of the end piece (1) and having a surface (8a) in contact with said base (5d) of the end piece,

    said burner being **characterized in that** said porous material is obtained from a composition comprising, as a percentage of the total weight of said composition, between 0 and 5% of at least one heat-conducting compound, with a thermal conductivity of greater than 60 watts per metre-kelvin, between 30% and 70% of at least one refractory compound, between 2% and 30% of at least one binder, and between 5% and 40% of at least one pore-forming agent, and
    **in that** all of said circular-shaped upper face (5c) is doped with said catalyst.

2.  Burner (10) according to Claim 1, in which at least a part of said inner face (5a) of said peripheral side wall (5) is doped with said catalyst.

3.  Burner (10) according to Claims 1 or 2, in which the lower end (61) of said inner face (5a) terminates with a counterbore (18) of diameter $\phi 3$, communicating with said cavity (2).

4.  Burner (10) according to any one of the preceding claims, in which the depth P of said second cavity (6) is between 2 and 8 mm, preferably between 6 and 7.5 mm and better still about 7 mm.

5.  Burner (10) according to any one of Claims 1 to 4, according to which said porous material is obtained from a composition comprising, as a percentage of the total weight of said composition, between 0.5% and 2% of said heat-conducting compound, between 40% and 70% of said refractory compound, between 5% and 25% of said binder, and between 8% and 35% of at least one pore-forming agent.

6.  Burner (10) according to any one of Claims 1 to 5, in which the heat-conducting compound is silicon carbide, preferably present in a proportion of 1% by weight relative to the total weight of said composition.

7.  Burner (10) according to any one of Claims 1 to 6, in which each said refractory compound is chosen from the group constituted of alumina, silica, mullite, zirconia and cordierite, and mixtures thereof, and preferably mullite.

8.  Burner (10) according to any one of Claims 1 to 7, in which said binder is a mineral compound which allows sintering at a temperature of less than or equal to 1100°C, preferably a glass, and better still a borosilicate glass.

9.  Burner (10) according to any one of Claims 1 to 8, in which the pore-forming agent is polymethyl methacrylate (PMMA), which is preferably present in a proportion of from 18% to 30% by weight relative to the total weight of said composition.

10. Burner (10) according to any one of Claims 6 to 9, according to which said porous material is obtained from a composition comprising, as a percentage of the total weight of said composition, about 1% of silicon carbide, between 60% and 70% of mullite, between 5% and 15% of glass, and between 18% and 30% of PMMA.

11. Burner (10) according to Claim 10, in which said sleeve (7) has a length of between 10 and 20 mm, and preferably about 14 mm.

12. Catalytic combustion bottle (20), which is suitable for containing a combustible liquid (30) and for receiving on its neck (50) a catalytic combustion burner (10) receiving a wick (40) which is soaking in said liquid (30), **characterized in that** said bottle (20) is equipped with a burner (10) as defined according to any one of Claims 1 to 11.

Fig. 1

Fig. 2

8

8a Fig. 3

8

Fig. 4

Fig. 5

Fig. 6

700,0°C

60,0°C

FIG. 7

700,0°C

— 600

— 400

— 200

60,0°C

FIG. 8

FIG. 9

FIG. 10

FIG. 11

FIG. 12

FIG. 13

FIG. 14

FIG. 15

FIG. 16

FIG. 17

FIG. 18

FIG. 19

## RÉFÉRENCES CITÉES DANS LA DESCRIPTION

**Documents brevets cités dans la description**

- FR 2610390
- FR 2095163 B1
- FR 2905164 B1
- FR 2905165 B1